Europäisches Patentamt

European Patent Office

Office européen des brevets

(11) Publication number: **0 357 428**
**A1**

# (12) EUROPEAN PATENT APPLICATION

(21) Application number: 89308827.8

(22) Date of filing: 31.08.89

(51) Int. Cl.⁵: **C 07 C 59/01**
**C 07 C 51/367**

(30) Priority: 01.09.88 JP 219140/88

(43) Date of publication of application:
07.03.90 Bulletin 90/10

(84) Designated Contracting States:
AT BE CH DE ES FR GB GR IT LI LU NL SE

(71) Applicant: WAKO PURE CHEMICAL INDUSTRIES LTD
1-2 Doshomachi-3-chome
Chuo-ku Osaka (JP)

(72) Inventor: Iwata, Tsutomu
775-3, Oka Myohoji
Suma-ku Kobe (JP)

Iwamoto, Osamu
10-24 Masumicho
Ikeda-shi (JP)

Sugiyama, Haruhiko
20-5 Onosuimei-1-chome Shigacho
Shiga-gun Shiga-ken (JP)

(74) Representative: Baillie, Iain Cameron et al
c/o Ladas & Parry Isartorplatz 5
D-8000 München 2 (DE)

(54) Process for preparing Beta-hydroxy fatty acid.

(57) β-Hydroxy fatty acid useful as a base material for lipids A, etc. is prepared by reacting an aliphatic aldehyde with a dianion of acetic acid.

EP 0 357 428 A1

**Description**

## PROCESS FOR PREPARING β-HYDROXY FATTY ACID

BACKGROUND OF THE INVENTION

This invention relates to a novel process for preparing β-hydroxy-fatty acids which are useful as base material for lipids A, LB films, cosmetics, liquid crystals and so on.

β-Hydroxy-fatty acids are a group of compounds which are attracting attention recently for their utility as a constituent of lipids A or as an important base material for LB films, cosmetics, liquid crystals and the like.

As a method for the synthesis of β-hydroxy-fatty acids, there is well known in the art a method making use of so-called Reformatzky reaction in which an aldehyde and an ester of bromoacetic acid are reacted in the presence of zinc. The reaction in this method, however, is violent and hard to control. Further, the yield of product is as low as only 10 to 12%, and many by-products are formed. A process for synthesizing the subject compounds by using a β-keto ester as intermediate, which process is higher in yield and more effective than said Reformatzky method, has also been reported (Acta Chem. Scand., 6, 809, 1952). This process, however, still has many problems in that it involves many steps (5 steps in all), the operation is troublesome, and the yield is also unsatisfactory: the overall yield from the starting fatty acids being 30 to 40%.

SUMMARY OF THE INVENTION

In view of the above, the present invention has been made with the object of providing a process for preparing β-hydroxy-fatty acids with a simple operation and in a high yield.

The present invention provides a process for preparing a β-hydryoxy-fatty acid represented by the formula:

$$CH_3(CH_2)_n\underset{\underset{OH}{|}}{C}HCH_2CO_2H \qquad (II)$$

wherein n represents an integer from 1 to 30, which comprises reacting an aliphatic aldehyde represented by the formula:

$$CH_3(CH_2)_nCHO \qquad (I)$$

wherein n represents the same as defined above, with a dianion of acetic acid.

DESCRIPTION OF THE PREFERRED EMBODIMENTS

Extensive studies have been made by the present inventors in search of a process capable of easy and high-yield preparation of β-hydroxy-fatty acids, and as a result, the present inventors found that the objective β-hydroxy-fatty acids can be obtained with a single step and in a remarkably higher yield by reacting an aliphatic aldehyde represented by the above-shown formula (I) with a dianion of acetic acid. This finding is the essence of the present invention.

The dianion of acetic acid used in the present invention can be easily obtained by reacting usually acetic acid or an alkaline metal salt thereof (such as sodium salt, potassium salt, lithium salt, etc.) with a basic substance having an activity necessary for dianionizing acetic acid (such basic substance being hereinafter referred to as "anionizing agent") in the presence of a solvent, usually at a temperature below 0°C.

Examples of the anionizing agents usable in this invention include metal amides such as sodium amide, potassium amide and lithium amide, lithium dialkyl amides such as lithium diethyl amide and lithium diisopropyl amide, lithium dicycloalkyl amides such as lithium dicyclohexyl amide, metallic hydrides such as sodium hydride and lithium hydride, alkyl or aryl lithiums such as methyl lithium, n-butyl lithium and phenyl lithium, 1,1,1,3,3,3-hexamethyldisilazane lithium salt, and the like.

Examples of the solvents usable in the above reaction according to this invention are ether type solvents such as tetrahydrofuran (THF), diethyl ether, dioxane and ethylene glycol dimethyl ether, and mixed solvents such as THF and n-hexane mixture, THF and n-heptane mixture, and the like.

The amount of anionizing agent used in the above reaction is usually 2 to 4 moles per mole of acetic acid when acetic acid is used in the reaction for obtaining a dianion of acetic acid, and 1 to 2 moles per mole of alkaline metal salt of acetic acid when such alkaline metal salt is used in said reaction. The present invention, however, is not limited thereto particularly.

When the anionizing agent used in the reaction is commercially available, such commercial product can be used without further purification. When the anionizing agent used is not commercially sold or hardly available, it may be prepared by a conventional method when required. For instance, in the case of lithium diisopropyl amide, it can be easily obtained by reacting, for example, n-butyl lithium and diisopropylamine with stirring at a temperature below 0°C in a solvent of the same type as used in said acetic acid dianionization reaction, and thus obtained lithium diisopropyl amide may be directly applied to the dianionization reaction.

The reaction between a dianion of acetic acid obtained in the manner described above and an aliphatic aldehyde represented by the formula (I) can be usually accomplished by adding an aliphatic aldehyde of the formula (I) directly into said acetic acid dianionization reaction solution and stirring the mixture. The reaction temperature is usually in the range from -30 to 50°C. The reaction time can be usually about 1 to 10 hours. In carrying out the reaction, if a solvent with extremely high polarity such as, for example, hexamethyl-phosphoric triamide (HMPA) is added for the purpose of raising solubility of a dianion of acetic acid, the reaction efficiency will be elevated to further increase the yield, so that the addition of such a solvent is desirable. In case of using HMPA, usually the amount thereof used is preferably about 5 to 15% by volume based on the amount of the whole reaction solution.

The reaction product is subjected to the adequate after-treatments, such as pH adjustment, solvent extraction, distilling-off of solvent, etc., for which the known methods can be used. If necessary, the product may be purified by suitable means such as column chromatography, recrystallization, etc.

According to the process of this invention, it is possible to obtain the desired β-hydroxy-fatty acid in a high yield in one pot and with substantially a single step, and further, the by-products are scarcely formed. Any of the compounds obtained according to the process of this invention can be easily optically resolved into R-form and S-form with an optical resolving agent, so that the process of this invention can also find its application for easy and simple preparation of optically active β-hydroxy-fatty acids.

The present invention will be described more particularly below by showing examples thereof, which examples, however, are merely intended to be illustrative and not to be construed as limiting the scope of the invention.

## EXAMPLE 1

Synthesis of 3-hydroxyoctanoic acid

40 g of diisopropylamine was dissolved in 260 ml of anhydrous THF and cooled to -20 to -25°C under a nitrogen stream. To this solution with stirring, there was added dropwise 180 ml of an n-hexane solution of n-butyl lithium (containing 15% of n-butyl lithium) at a temperature below 0°C, followed by further addition thereto of a solution of 10 g of acetic acid in 15 ml of anhydrous THF with stirring at a temperature below 0°C. Then, after addition of 36 ml of HMPA, the mixture was stirred at room temperature for 30 minutes, quickly added with 17.0 g of 1-hexanal at a temperature below 0°C and reacted at room temperature for 2.5 hours. The resulting reaction solution was added with 600 ml of a 10% hydrochloric acid solution and extracted with chloroform. The chloroform layer was washed with water and dried over anhydrous $Na_2SO_4$ and then the solvent was distilled off. The resulting syrup was purified by column chromatography (filler: Wako Gel C-300 (trade name, manufactured by Wako Pure Chemical Industries, Ltd.); eluent: ethyl acetate/n-hexane (1/5)) to obtain 18.5 g of the objective colorless needle crystals.
Yield: 68%; m.p.: 34 - 35°C.

Elemental analysis, calcd. for $C_8H_{16}O_3$

| | |
|---|---|
| Theoretical: | C, 59.97; H, 10.07 (%) |
| Found: | C, 59.91; H, 10.02 (%) |

IR (neat) ν max cm$^{-1}$: 3600 - 2800 (OH, COOH), 1705 (COOH). NMR (CDCl$_3$) δ ppm: 0.83 (t-like, 3H, CH$_3$), 1.10 - 1.92 (m, 8H, CH$_2$×4), 2.40 - 2.67 (m, 2H, CH$_2$-CO$_2$H), 3.83 - 4.33 (m, 1H, CH), 6.97 (s, 2H, COOH, OH).

## EXAMPLE 2

Synthesis of 3-hydroxytridecanoic acid

56 g of diisopropylamine was dissolved in 360 ml of anhydrous THF and cooled to -20 to -25°C under a nitrogen stream. To this solution with stirring, there was added dropwise 250 ml of an n-hexane solution of n-butyl lithium (containing 15% w/v of n-butyl lithium) at a temperature below 0°C, followed by further addition thereto of a solution of 14 g of acetic acid in 20 ml of anhydrous THF with stirring at a temperature below 0°C. Then 50 ml of HMPA was added and the solution was stirred at room temperature for 30 minutes. Thereafter, 50 ml of 1-undecanal was added quickly at a temperature below 0°C and the mixture was stirred and reacted at room temperature for 2.5 hours. The resulting reaction solution was added with 800 ml of a 10% hydrochloric acid solution and extracted with chloroform. The chloroform layer was washed with water and dried over anhydrous $Na_2SO_4$. Then the solvent was distilled off and the residue was recrystallized from n-hexane to obtain 34.8 g of the objective colorless needle crystals having a melting point of 81 - 82°C. Yield was 65%.

Elemental analysis, calcd. for $C_{13}H_{26}O_3$

| | |
|---|---|
| Theoretical: | C, 67.78; H, 11.38 (%) |
| Found: | C, 67.72; H, 11.24 (%) |

IR (KBr) ν max cm$^{-1}$: 3540 (OH), 3400 - 2800 (COOH), 1680 (COOH).

## EXAMPLES 3 - 13

The following Compound Nos. 3 - 13 were synthesized in the same way as Example 1 or 2. The yields and melting points of the obtained compounds are also shown in Table 1.

3

Table 1

| No. | Compound name | Compositional formula | Yield | m.p. |
|---|---|---|---|---|
| 3 | 3-hydroxynonanoic acid | $C_9H_{18}O_3$ | 62% | 57.5 ~ 58.5°C |
| 4 | 3-hydroxydecanoic acid | $C_{10}H_{20}O_3$ | 66% | 56 ~ 57°C |
| 5 | 3-hydroxyundecanoic acid | $C_{11}H_{22}O_3$ | 58% | 70 ~ 71°C |
| 6 | 3-hydroxydodecanoic acid | $C_{12}H_{24}O_3$ | 60% | 68 ~ 69°C |
| 7 | 3-hydroxytetradecanoic acid | $C_{14}H_{28}O_3$ | 67% | 77 ~ 78°C |
| 8 | 3-hydroxypentadecanoic acid | $C_{15}H_{30}O_3$ | 55% | 86.5 ~ 87°C |
| 9 | 3-hydroxyhexadecanoic acid | $C_{16}H_{32}O_3$ | 61% | 83 ~ 84°C |
| 10 | 3-hydroxyheptadecanoic acid | $C_{17}H_{34}O_3$ | 58% | 90 ~ 91°C |
| 11 | 3-hydroxyoctadecanoic acid | $C_{18}H_{36}O_3$ | 54% | 89 ~ 90°C |
| 12 | 3-hydroxynonadecanoic acid | $C_{19}H_{38}O_3$ | 60% | 95 ~ 96°C |
| 13 | 3-hydroxyeicosanoic acid | $C_{20}H_{40}O_3$ | 62% | 92 ~ 93°C |

The present invention provides a novel and notably simple process for the preparation of β-hydroxy-fatty acids which can be an important base material for lipids A, Lb films, cosmetics, liquid crystals, etc. According to the process of this invention, it is possible to obtain a desired β-hydroxy-fatty acid in one pot with a single step and in a high yield, and further, few by-products are formed. Thus, the present invention has its particularly remarkable effect in making it possible to produce β-hydroxy-fatty acids on an industrial scale.

**Claims**

1. A process for preparing a β-hydroxy-fatty acid represented by the formula:

$$CH_3(CH_2)_n \underset{\underset{OH}{|}}{CH} CH_2CO_2H$$

wherein n represents an integer from 1 to 30, which comprises reacting an aliphatic aldehyde represented by the formula:

$$CH_3(CH_2)_nCHO$$

wherein n represents the same as defined above, with a dianion of acetic acid.

2. The process according to Claim 1, wherein said dianion of acetic acid is the one obtained by reacting acetic acid or an alkaline metal salt thereof with sodium amide, potassium amide, lithium amide, lithium diethyl amide, lithium diisopropyl amide, lithium dicyclohexyl amide, sodium hydride, lithium hydride, methyllithium, n-butyl lithium, phenyllithium or 1,1,1,3,3,3-hexamethyldisilazane lithium salt.

3. The process according to Claim 1, wherein the reaction is carried out in the presence of hexamethyl-phosphoric triamide.

4. The process according to Claim 3, wherein the hexamethylphosphoric triamide is present in an amount of 5 to 15% by volume in the reaction solution.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | BULLETIN DE LA SOCIETE CHIMIQUE DE FRANCE, no. 5, May 1970, pages 1848-1849; B. ANGELO: "No 316. - Synthèse directe d'acides beta-hydroxylés, par l'intermédiaire des systèmes "hydrocarbures aromatiques-métaux alcalins"" <br> * Whole article * <br> ----- | 1-2 | C 07 C 59/01 <br> C 07 C 51/367 |

TECHNICAL FIELDS
SEARCHED (Int. Cl.5)

C 07 C 59/00
C 07 C 51/00

The present search report has been drawn up for all claims

| Place of search | Date of completion of the search | Examiner |
|---|---|---|
| THE HAGUE | 24-11-1989 | KLAG M.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)